Europäisches Patentamt

(19) European Patent Office

Office européen des brevets

(11) Publication number: **0 076 376**
A1

(12) **EUROPEAN PATENT APPLICATION**

(21) Application number: 82107558.7

(22) Date of filing: 18.08.82

(51) Int. Cl.³: **C 07 C 149/12**

(30) Priority: 05.10.81 US 308328

(43) Date of publication of application:
13.04.83 Bulletin 83/15

(84) Designated Contracting States:
BE CH DE FR GB IT LI NL SE

(71) Applicant: PENNWALT CORPORATION
Pennwalt Building Three Parkway
Philadelphia Pennsylvania 19102(US)

(72) Inventor: Dimmig, Daniel Ashton
300 Riverview Road
King of Prussia Pennsylvania 19406(US)

(74) Representative: Kraus, Walter, Dr. et al,
Patentanwälte Dres. Kraus & Weisert Irmgardstrasse 15
D-8000 München 71(DE)

(54) Deodorizing dialkyl polysulfides.

(57) A method is disclosed for improving the odor of dialkyl polysulfides of the formula $R-S_xR^1$ (R and $R^1$ are $C_1-C_{18}$ alkyl or substituted-alkyl groups, x is 2-6) which contain sulfur bearing odorriferous impurities wherein the impure polysulfide is intimately mixed with a specified metal salt for a period of time sufficient to reduce the odor of the mixture.

EP 0 076 376 A1

Croydon Printing Company Ltd

## Deodorizing Dialkyl Polysulfides

## Background Of The Invention

Dialkyl polysulfides having the general formula $R-S_x-R^1$ wherein R and $R^1$ are $C_1$ to $C_{18}$ alkyl or substituted-alkyl groups and x is an integer of from 2 to 6 are of considerable commercial importance as additives for extreme pressure lubricants and cutting oils. The substituted-alkyl groups may be, for example, haloalkyl, hydroxyalkyl, or alkoxyalkyl. A convenient and economical process for the production of the dialkyl polysulfides involves the reaction of alkyl or substituted-alkyl mercaptans with elemental sulfur in the presence of a basic catalyst to form mixtures of dialkyl polysulfides having sulfur chain lengths that vary according to the ratio of reactants employed. Polysulfides produced in accordance with the above described reaction are characterized by unpleasant odors resulting from the presence of trace amounts of hydrogen sulfide, unreacted alkyl mercaptans and other impurities.

## Statement Of The Invention

This invention concerns contacting dialkyl polysulfides of the general formula $R\text{-}S_x\text{-}R^1$ wherein R and $R^1$ are primary, secondary or tertiary alkyl or substituted-alkyl (e.g. haloalkyl, hydroxyalkyl or alkoxyalkyl) groups having from 1 to 18 carbon atoms and x is an integer of from 2 to 6, said polysulfides being contaminated with traces of odoriferous sulfur-bearing impurities as a result of the process of preparing said polysulfides, with an inorganic or $C_1$-$C_{18}$ saturated monocarboxylic acid salt of a metal selected from the group consisting of manganese, iron, cobalt, nickel, copper, zinc, silver, lead, cadmium and mercury in amount of from about 0.001 to about 0.04 gram-mole of salt for each 100 grams of polysulfide, and, while agitating, maintaining the temperature of the mixture within the range of ambient to about 85°C for a time sufficient to reduce the intensity of the odor of said contaminated polysulfide.

This invention also comprises a mixture of the above described salts and the dialkyl polysulfide as well as the dialkyl or di(substituted alkyl) polysulfide containing product of the above described process, particularly that product which has been subjected to filtration after processing.

The following examples are set forth to demonstrate this invention:

## Example 1

A mixture of 150 grams of di-tertiary-nonyl polysulfide having an average sulfur chain length from about 4.5 to 5 and contaminated with trace amounts of sulfur-bearing impurities including hydrogen sulfide, unreacted alkyl mercaptans and other substances resulting from the preparation of said polysulfide by the basic catalyzed reaction of t-nonyl mercaptan with elemental sulfur, and 0.75 gram of anhydrous cupric sulfate (powder) was charged to a 250 ml. round-bottomed flask. The mixture was warmed to 65°C and stirred at this temperature for one hour. The mixture was then filtered and subjected to an odor evaluation as hereinafter described. The data for the above procedure and evaluation are reported in Table 1.

## Examples 2-4

The above procedure was repeated several times except that different amounts of anhydrous cupric sulfate (powder) and different periods of heating the stirred mixture were employed. The data for these procedures and evaluations are reported in Table 1.

## Examples 5-7

On a larger scale, a mixture of 458 pounds of di-t-nonyl polysulfide having a sulfur chain length average and contamination as described in Example 1 and 14 pounds of powdered cupric sulfate monohydrate were charged to a 60-gallon stainless steel vessel. The mixture was warmed to 60°C and agitated with a flat-blade, turbine-type stirrer at 60-65°C for a total of six hours. Samples were taken for odor panel evaluation at two hour intervals. The data for the above procedures and evaluation are reported in Table 1.

## Example 8

In another experiment, the procedure of Example 1 above was repeated except that the copper salt was replaced with ferric chloride in the amount of 1 percent based on the weight of the mixture. The data for this procedure are reported in Table 1.

The odor of each of the treated polysulfide samples and a control (untreated polysulfide of Examples 1-4) were evaluated by a panel of at least ten persons by smelling the untreated and treated products and observing the intensity of the odor and the degree of like or dislike of the smell (odor type). The evaluation results of these panelists were tabulated and averaged to produce the data of the table. Odor intensity was rated in accordance with the following system:

| Rating | - | Intensity |
|--------|---|-----------|
| 1 | | no odor |
| 2 | | trace |
| 3 | | mild |
| 4 | | moderate |
| 5 | | strong |

The degree of like or dislike (odor type) was classified as follows:

| Rating | Classification |
|--------|----------------|
| +3 | like very much |
| +2 | like moderately |
| +1 | like slightly |
| 0 | neither like or dislike |
| -1 | dislike slightly |
| -2 | dislike moderately |
| -3 | dislike very much |

Table 1

| Example No. | Metal Salt | | Time, Hrs.** | Odor Evaluation | |
|---|---|---|---|---|---|
| | Wt.% | Mole% | | Intensity | Type |
| Control | 0 | 0 | 0 | 3.4 | -1.6 |
| 1 | 0.5 | 0.0031 | 1 | 3.5 | -1.5 |
| 2 | 0.5 | 0.0031 | 6 | 2.8 | +0.2 |
| 3 | 1.0 | 0.0062 | 4 | 2.6 | -0.3 |
| 4 | 1.0 | 0.0062 | 6 | 2.3 | -0.3 |
| 5 | 3.0 | 0.0169 | 2 | 2.9 | -0.9 |
| 6 | 3.0 | 0.0169 | 4 | 2.3 | -0.2 |
| 7 | 3.0 | 0.0169 | 6 | 2.6 | -0.1 |
| 8 | 1.0 | 0.0062 | 6 | 2.5 | -0.3 |

* - Percent of salt in the mixture of salt and contaminated polysulfide.

** - Number of hours mixture is subjected to elevated temperature.

The data of the above table show a reduction of odor intensity and an improvement in odor type in the process of this invention for those treated polysulfide samples in which sufficient time was employed for heating the mixture. Heating was continued in Example 1 for only one hour, which proved to be too little for the particular contaminated polysulfide treated, while the same contaminated material and salt mixture, when treated for six hours (Example 2), had significantly reduced odor intensity and improved odor type.

## Discussion of the Invention

This invention concerns the substantial deodorization of di($C_1$-$C_{18}$) alkyl or substituted alkyl polysulfide ($S_2$-$S_6$) preparations containing, as a result of their manufacture, e.g., reaction between an alkyl mercaptan and elemental sulfur, odoriferous sulfur-bearing contaminents. The treated polysulfides have the general formula $R$-$S_x$-$R^1$ where R and $R^1$ are primary, secondary or tertiary alkyl or substituted-alkyl (e.g. haloalkyl, hydroxyalkyl or alkoxyalkyl) groups having from 1 to 18 carbon atoms and x is an integer of from 2 to 6. The polysulfides are usually formed as a mixture of several components meeting the above formula and preferably consist of polysulfides wherein the alkyl groups are tertiary alkyl groups having from 8 to 12 carbon atoms and each such alkyl substituent of the dialkyl polysulfide is the same, i.e., R and $R^1$ are identical. The polysulfide chain portion of the molecule will preferably average from 3 to 5 sulfur atoms, i.e., in the formula $R$-$S_x$-$R^1$, x averages from 3 to 5. The most preferred dialkyl polysulfides for this invention are tertiary-nonyl trisulfide and tertiary-nonyl polysulfide which is a mixture of polysulfides of the formula $R$-$S_x$-$R^1$ wherein x has an average value ranging from about 4.5 to 5.0. A preferred di-substituted alkyl polysulfide is bis(2-hydroxyethyl) disulfide.

The metal salts which are used in this invention are hydrated or anhydrous inorganic or $C_1$-$C_{18}$ saturated monocarboxylic acid salts of metals selected from the group consisting of manganese, iron, cobalt, nickel, copper, zinc, silver, lead, cadmium and mercury. The anions of said salts are residues of inorganic acids e.g. sulfate, chloride, nitrate, nitrite, borate, bromide, phosphate and carbonate or of $C_1$-$C_{18}$ saturated monocarboxylic acids, e.g., acetate, butyrate, caprylate, laurate, and stearate. Preferred salts are those formed with copper, particularly copper sulfate.

The metal salts are used in an amount of from about 0.001 to about 0.04 gram-mole of salt per hundred grams of the contaminated polysulfide, preferably from about 0.0025 to about 0.025 gram-mole per hundred grams of polysulfide. During the treatment of the mixture, it is agitated to ensure intimate contact of the ingredients.

The temperature at which the polysulfide-salt mixture is maintained during the process ranges from about ambient to about 85°C, preferably from about 50 to about 70°C.

The minimum time that the process is carried out is about two hours and during this minimum time duration the mixture should be heated to at least the minimum temperature of the preferred temperature range and the mixture should contain an amount of salt at least equivalent to the minimum amount of salt required in the preferred range. Where the process is carried out at elevated temperature, heating is

0076376

continued for from about 2 to 10 hours, preferably from about 3 to about 6 hours while heating within the preferred temperature range. At ambient temperature, agitation of the mixture is usually continued for at least 6 hours up to 10 hours or longer.

Good agitation of the mixture, ensuring intimate contact of the metal salt and the contaminents of the dialkyl polysulfide preparation, is beneficial for reducing odor more efficiently. The use of the metal salt in a finely powdered state wherein the powder has a high surface area also helps provide good contact of the components of the mixture.

After carrying out the treatment, the contaminants of the polysulfide preparation, including the process catalyst, metal sulfides, mercaptides or mercaptans, and any excess metal salt are preferably substantially removed by filtration.

## Claims

1. The method of treating liquid dialkyl polysulfides of the general formula $R-S_xR^1$ wherein R and $R^1$ are alkyl or substituted-alkyl groups having from 1 to 18 carbon atoms and x is an integer of from 2 to 6, said polysulfides being contaminated with traces of odoriferous sulfur-bearing impurities as a result of the process of manufacturing said polysulfides, said method comprising contacting said contaminated dialkyl polysulfide with an inorganic or a $C_1-C_{18}$ saturated monocarboxylic acid salt of a metal selected from the group consisting of manganese, iron, cobalt, nickel, copper, zinc, silver, lead, cadmium and mercury, in an amount of from about 0.001 to about 0.04 gram-mole of salt for each 100 grams of polysulfide, and, while agitating to provide an intimate mixture of salt and polysulfide, maintaining the temperature of the mixture within the range of ambient to about 85°C for a time sufficient to reduce the intensity of the odor of said contaminated polysulfide.

2. The method of claim 1 wherein x has an average value of from 3 to 5, R and $R^1$ are tertiary alkyl groups having from 8 to 12 carbon atoms and R and $R^1$ are the same.

3. The method of claim 1 wherein said polysulfide is bis (2-hydroxethyl) disulfide.

4.    The method of claim 2 wherein the metal of the salt is copper and the salt is used in an amount ranging from about 0.0025 to about 0.025 gram-mole for each hundred grams of contaminated polysulfide.

5.    The method of claim 2 wherein the metal of the salt is iron and the salt is used in an amount ranging from about 0.0025 to about 0.025 gram-mole for each hundred grams of contaminated polysulfide.

6.    The method of claim 4 wherein the metal salt is copper sulfate.

7.    The method of claim 1, 2, 3, 4, 5, or 6 wherein the temperature of the mixture is maintained within the range of about 50 to about 70°C for a period of time ranging from about 2 to about 10 hours.

8.    The method of claim 1 wherein the dialkyl polysulfides are mixtures of compounds meeting the general formula $R-S_x-R^1$ where R and $R^1$ are tertiary nonyl groups and x has an average value of 4.5 to 5, the salt is copper sulfate in an amount of from about 0.0025 to about 0.025 gram-mole per hundred grams of contaminated polysulfide and the temperature is maintained within the range of about 50 to about 70°C for a period of time ranging from about 3 to about 6 hours.

9.  The method of claim 1 wherein the treatment is followed by a filtration step to remove contaminants from said polysulfides.

10.  The method of claim 8 wherein the treatment is followed by a filtration step to remove contaminants from said polysulfides.

11.  The product of the method of claim 9.

12.  The product of the method of claim 10.

**0076376**

Application number

# EUROPEAN SEARCH REPORT

European Patent Office

EP 82 10 7558

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | C 07 C 149/12 |
| X | US-A-3 340 324 (PAUL F.WARNER) *Column 5, lines 69-73* | 1,4 | |
| | --- | | |
| A | CHEMICAL ABSTRACTS, vol. 79, no. 26, 31st December 1973, page 170, no. 149180x, Columbus Ohio (USA); & JP - A - 73 52 671 (NIPPON SYN-THETIC CHEMICAL INDUSTRY) (24-07-1973) *Abstract* | 1 | |
| | ----- | | |

TECHNICAL FIELDS SEARCHED (Int. Cl. ³)

C 07 C 149/00

The present search report has been drawn up for all claims

| Place of search | Date of completion of the search | Examiner |
|---|---|---|
| THE HAGUE | 11-11-1982 | GRAMAGLIA R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82